(19) Europäisches Patentamt
European Patent Office
Office européen des brevets

(11) **EP 4 779 014 A1**

(12) **EUROPEAN PATENT APPLICATION**
published in accordance with Art. 153(4) EPC

(43) Date of publication:
**22.07.2026 Bulletin 2026/30**

(21) Application number: **24865519.3**

(22) Date of filing: **12.09.2024**

(51) International Patent Classification (IPC):
***C12N 5/00*** *(2006.01)*

(52) Cooperative Patent Classification (CPC):
**C12N 5/00**

(86) International application number:
**PCT/JP2024/032716**

(87) International publication number:
**WO 2025/058019 (20.03.2025 Gazette 2025/12)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **15.09.2023 JP 2023150360**

(71) Applicants:
• **Kuraray Co., Ltd.
Kurashiki-shi, Okayama 710-0801 (JP)**
• **Tabata, Yasuhiko
Uji-shi
Kyoto 611-0024 (JP)**

(72) Inventors:
• **SHIRAI, Takanori
Kurashiki-shi, Okayama 713-8550 (JP)**
• **OKADA, Chieko
Kurashiki-shi, Okayama 713-8550 (JP)**
• **OCHIAI, Toru
Okayama-shi, Okayama 702-8045 (JP)**
• **TABATA, Yasuhiko
Uji-shi, Kyoto 611-0024 (JP)**

(74) Representative: **Müller-Boré & Partner
Patentanwälte PartG mbB
Friedenheimer Brücke 21
80639 München (DE)**

(54) **CELL AGGREGATE AND METHOD FOR PRODUCING CELL AGGREGATE**

(57)    Provided are a cell aggregate comprising adherent cells and an extracellular matrix, the cell aggregate containing living cells even in a part located at a depth of 30 μm or more from the surface of the aggregate; and a method for producing a cell aggregate, the method comprising supporting adherent cells on a molded body formed by fibers containing an ethylene-vinyl alcohol copolymer and having a nonwoven fiber structure and culturing the cells while stirring.

**EP 4 779 014 A1**

## Description

TECHNICAL FIELD

The present invention relates to a cell aggregate comprising an adherent cell and an extracellular matrix, and a method for producing the cell aggregate.

BACKGROUND ART

In recent years, in order to sufficiently exert the function of transplanted cells in cell transplantation therapy, increasing attention has been directed to transplantation of cell aggregates (also referred to as cell clumps or cell spheroids) produced by three-dimensional culture, which better reflect in vivo intracellular interactions and cell-extracellular matrix interactions, rather than single cells cultured in a monolayer.

Methods for producing cell aggregates have been previously reported, and representative examples thereof include a method comprising culturing cells using a culture plate having a non-adhesive bottom surface and adhering cells floating in a medium to each other to produce a cell aggregate (the non-adhesive surface cell culturing method), a method comprising dropping a cell suspension inside a lid of a culture plate and culturing droplets formed in a dome shape by surface tension upside down to gather cells in a dripping manner by gravity, thereby adhering the cells to each other to form a cell aggregate (the hanging drop cell culturing method), and a method comprising culturing cells by rotating a cell culture chamber and bringing cells into contact with each other in the chamber to form a cell aggregate (the rotary cell culturing method). Furthermore, collagen-rich cell aggregates of mesenchymal stem cells produced in a growth medium to which factors for producing collagen are added, and the cryopreserved transplants for mesenchymal stem cell transplantation obtained by cryopreserving the cell clumps with cryopreservation media have been reported (Patent Document 1).

PRIOR ART DOCUMENT

PATENT DOCUMENT

Patent Document 1: JP-B-7105487

SUMMARY OF THE INVENTION

PROBLEMS TO BE SOLVED BY THE INVENTION

In cell transplantation therapy using cell aggregates, it is necessary to prepare cell aggregates every time transplantation surgery is performed, but there is no guarantee that the number of cells and cell functions of the cell aggregate can be made uniform. On the other hand, if cell aggregates satisfying a certain quality is cryopreserved and thawed for transplantation at the time of transplantation surgery, a stable transplantation effect can be expected, but in the prior art, the cell aggregate exhibits low cell viability after thawing, and therefore it is difficult to cryopreserve the cell aggregates. Therefore, an object of the present invention is to provide cryopreservable cell aggregates and a production method for the cell aggregates.

SOLUTIONS TO THE PROBLEMS

As a result of intensive studies, the present inventors surprisingly have succeeded in obtaining cell aggregates by supporting adherent cells on a shaped product made of fibers containing ethylene-vinyl alcohol-based copolymers, the shaped product having a nonwoven fiber structure, and culturing the adherent cell under stirring. Further surprisingly, it has been found that the cell aggregate exhibits a high cell survival rate, with cells in the central part of the cell aggregate remaining viable even after freezing and thawing the cell aggregate, and is thus capable of cryopreservation. Thus, the present invention has been completed.

That is, the present invention provides the followings.

(1) A cell aggregate comprising adherent cells and extracellular matrix molecules, the cell aggregate comprising viable cells even at a depth of 30 $\mu$m or more from a surface of the cell aggregate.

(2) The cell aggregate according to (1), comprising viable cells even at a depth of 30 $\mu$m or more from the surface of the cell aggregate after freezing and thawing the cell aggregate.

(3) The cell aggregate according to (1) or (2), which is granular with a diameter of more than 1.5 mm.

(4) The cell aggregate according to any one of (1) to (3), wherein less than 50% of the extracellular matrix molecules is collagen.

(5) The cell aggregate according to any one of (1) to (4), which is in a frozen state.

(6) A method for producing a cell aggregate, the method comprising:

supporting an adherent cell on a shaped product made of a fiber containing an ethylene-vinyl alcohol-based copolymer, the shaped product having a nonwoven fiber structure; and culturing the adherent cell under stirring.

(7) The method according to (6), wherein the fiber containing an ethylene-vinyl alcohol-based copolymer is a thermal adhesive fiber under moisture.

(8) The method according to (6), wherein the fiber containing an ethylene-vinyl alcohol-based copolymer is a composite fiber containing a non thermal adhesive fiber under moisture.

(9) The method according to (8), wherein the composite fiber is a core-sheath type composite fiber comprising a sheath part comprising an ethylene-vinyl alcohol-based copolymer and a core part comprising a polyester-based resin.
(10) The method according to any one of (6) to (9), comprising:

supporting the adherent cell on the shaped product having an apparent density of 0.02 to 0.7 g/cm3: and
culturing the adherent cell under stirring at 30 to 140 rpm.

EFFECTS OF THE INVENTION

According to the present invention, a cell aggregate is obtained by a simple method, the method comprising supporting an adherent cell on a shaped product composed of a fiber containing an ethylene-vinyl alcohol-based copolymer and having a nonwoven fiber structure, and culturing the adherent cell under stirring. The cell aggregates obtained by the present invention have larger size, improved cell viability during culture, and improved cell viability after freeze-thawing, as compared to cell aggregates obtained by the prior art. The cell aggregate of the present invention is a cryopreservable cell aggregate because cells in the central part of the cell aggregate remain viable even after freezing and thawing the cell aggregate. Thus, when the cell aggregate of the present invention is used, it is not necessary to prepare the cell aggregate for each therapy, and a lead time of cell transplantation therapy (a time from determination of therapy to start of therapy) is shortened.

BRIEF DESCRIPTION OF THE DRAWINGS

[Fig. 1] Fig. 1 provides photomicrographs showing results of Live-Dead assay of cell aggregates.
[Fig. 2] Fig. 2 provides photomicrographs showing results of Live-Dead assay of cell aggregates after freeze-thawing.
[Fig. 3] Fig. 3 provides photomicrographs showing results of picro-Sirius red staining of cell aggregates.

MODE FOR CARRYING OUT THE INVENTION

1. Cell Aggregate

As used herein, the term "cell aggregate" refers to a mass of cells formed by gathering or aggregating a plurality of cells. The cell aggregate of the present invention is an aggregate of adherent cells, and comprises an extracellular matrix in addition to the cells. The cell aggregate of the present invention is a cell aggregate produced in vitro or ex vivo.
As used herein, the term "adherent cell" refers to a cell that proliferates by adhering to a culture carrier during cell culture. The adherent cell may be derived from any organism such as an animal (for example, mammalian or non-mammalian), a plant, or an insect, and the origin of the adherent cell is not particularly limited. Examples of the adherent cell include, but are not limited to, an osteoblast, a chondrocyte, a hematopoietic cell, an epithelial cell (for example, a mammary epithelial cell or the like), an endothelial cell (for example, a vascular endothelial cell or the like), an epidermal cell, a fibroblast, a mesenchymal-derived cell, a cardiomyocyte, a myogenic cell, a smooth muscle cell, a biologically-derived skeletal muscle cell, a human tumor cell, a fibrous cell, an EB virus mutant cell, a hepatocyte, a renal cell, a bone marrow cell, a macrophage, a hepatocyte cell, a pancreatic β cell, a small intestine cell, a mammary gland cell, a salivary gland cell, a thyroid cell, a skin cell, and a somatic stem cell (for example, a mesenchymal stem cell or the like).
As used herein, the term "extracellular matrix molecule" refers to a non-cellular component which is an insoluble substance present extracellularly in an organism and fills spaces between cells. The extracellular matrix is mainly composed of water, proteins, and polysaccharides and is known that the composition and form of the extracellular matrix differ depending on the kinds of biological tissue. The extracellular matrix molecules contained in the cell aggregate of the present invention may preferably be extracellular matrix molecules produced by the adherent cells themselves contained in the cell aggregate. The cell aggregate of the present invention contains a relatively small amount of collagen as the extracellular matrix molecules, and for example, less than about 50%, preferably about 48% or less, more preferably about 46% or less of the extracellular matrix molecules contained in the cell aggregate of the present invention may be collagen.
The cell aggregate of the present invention has, for example, a granular shape. The size of the granular cell aggregate may be, for example, about 1.5 mm or more, preferably about 2 mm or more, more preferably about 5 mm or more in diameter (if the cell aggregate is not a sphere, the longest diameter, hereinafter referred to as a "major diameter"). The cell aggregate of the present invention may be, for example, a granular aggregate having a major diameter of about 1.5 mm to about 5 mm. The diameter of the cell aggregate may be measured by a conventional method, and can be measured using, for example, an observation image of a microscope.
Furthermore, in the cell aggregate of the prior art, the cells in the central part of the aggregate die after freezing and thawing the cell aggregate, whereas in the cell aggregate of the present invention, the cells in the central part of the aggregate remain viable even after freezing and thawing the aggregate, and the cell viability after freezing and thawing the aggregate is improved as compared with the prior art. In addition, in the cell aggregate of the prior art, when the size is large, for example with a diameter of more than about 1.5 mm, the cells in the central part of the aggregate die even without undergoing freeze-thawing. In contrast, in the cell aggregate of the present invention, the cells in the central part of the aggregate remain viable even when the size is large, and the cell viability is improved as compared with the prior art.

As used herein, the central part refers to, for example, a region at a depth of about 30 $\mu$m or more, preferably about 40 $\mu$m or more from the surface of the cell aggregate (that is, the interior region of the cell aggregate at least about 30 $\mu$m, preferably at least about 40 $\mu$m away from the surface of the cell aggregate), depending on the size of the cell aggregate. The distance from the surface to the depth of the cell aggregate can be measured using, for example, a microscopic image. In addition, the survival of cells in the cell aggregate can be confirmed by microscopic observation. Thus, the cell aggregate of the present invention is a cryopreservable cell aggregate, i.e., freeze-thawable cell aggregate.

Accordingly, the cell aggregate of the present invention may be a cell aggregate in a frozen state. The cell aggregate is preferably frozen in a cryopreservation solution. As the cryopreservation solution, a cryopreservation solution known in the art, for example a cryopreservation solution containing 10% dimethyl sulfoxide (DMSO), 20% fetal bovine serum (FBS), and 70% Dulbecco's Modified Eagle Medium (DMEM), or a commercially available cryopreservation solution may be used. Examples of the commercially available cryopreservation solution include, but not limited to, CELLBANKER 1 (manufactured by Zenogen Pharma Co., Ltd.). The cell aggregate may be frozen at a temperature at which freezing can be performed, for example, 0°C or less, -70°C or less, or -80°C or less. The cell aggregate may further be stored in a frozen state, for example, but not limited to, for 24 hours or more, or even for a long period of time of 1 month or more. Freezing and cryopreservation may be performed in a freezer or in liquid nitrogen. The freezing temperature and the cryopreservation temperature may be the same or different. The freezing temperature may be any temperature as long as freezing is possible, and is not particularly limited. The cryopreservation temperature may be any temperature as long as a frozen state can be maintained, and is not particularly limited. The freezing temperature, the cryopreservation temperature, and the storage period may be appropriately selected by those skilled in the art. For example, the cell aggregate may be placed in a freezer at about -70°C to -90°C for about 24 hours to 72 hours, then transferred into liquid nitrogen at about -196°C, and stored for 24 hours or more.

The cell aggregate of the present invention is used, for example, as a cell transplant, and also used in a test using cells, such as screening for an anticancer agent. Particularly, since the cell aggregate of the present invention can be frozen and thawed, the cell aggregate of the present invention may be prepared and stored in advance before use, offering high convenience.

2. Production Method for Cell Aggregate

The cell aggregate of the present invention can be produced by a method comprising supporting an adherent cell on a shaped product composed of a fiber containing an ethylene-vinyl alcohol-based copolymer and having a nonwoven fiber structure, and culturing the adherent cell under stirring (hereinafter, referred to as "the production method of the present invention"). According to the production method of the present invention, the adherent cells proliferate within voids of the shaped product, the cells gather or aggregate with each other to form cell aggregates, and the cell aggregates are then naturally separated from the shaped product. Further, the stirring promotes the proliferation of the cells and the separation of the cell aggregates. Specifically, the stirring promotes the supply of oxygen and nutrients necessary for cell survival into the nonwoven fiber structure, thereby promoting the cell proliferation. The promotion of cell proliferation then provides the number of cells necessary for the formation of cell aggregates inside the nonwoven fiber structure, leading to the promotion of cell aggregate formation. Furthermore, the stirring has the effect of physically separating the cell aggregates formed in the nonwoven fiber structure from the nonwoven fiber structure. As used herein, the fiber containing an ethylene-vinyl alcohol-based copolymer is preferably a thermal adhesive fiber under moisture.

As used herein, the "nonwoven fiber structure" refers to a board-like structure formed by bonding fibers without weaving.

As used herein, the term "thermal adhesive fiber under moisture" refers to a fiber that contains a thermal adhesive resin under moisture and exhibits an adhesive function under high-temperature steam or hot water. As used herein, the term "thermal adhesive resin under moisture" refers to a resin that is softened under high-temperature steam or hot water to exhibit an adhesive function.

Examples of the thermal adhesive resin under moisture include a thermoplastic resin that is capable of self-adhering or adhering to other fibers when softened by hot water (for example, about 80 to 120°C, particularly about 95 to 100°C), particularly a hydrophilic resin and a water-soluble resin. Specific examples thereof include a cellulose-based resin (C1-3 alkyl cellulose ether such as methyl cellulose, a hydroxy C1-3 alkyl cellulose ether such as hydroxymethyl cellulose, a carboxy C1-3 alkyl cellulose ether such as carboxymethyl cellulose, or a salt thereof, and the like), a polyalkylene glycol resin (poly C2-4 alkylene oxide such as polyethylene oxide and polypropylene oxide, and the like), a polyvinyl-based resin [polyvinyl pyrrolidone, polyvinyl ether, vinyl alcohol polymer (vinyl alcohol-based polymer containing $\alpha$-C2-10 olefin units such as ethylene and propylene, particularly ethylene-vinyl alcohol-based copolymer), polyvinyl acetal, and the like], an acryl-based copolymer and a salt thereof [a copolymer containing a unit composed of an acrylic monomer such as (meth)acrylic acid and a salt thereof, and (meth)acrylamide, particularly a (meth)acrylic copolymer containing a (meth)acrylamide unit, and the like], a modified vinyl-based copolymer (a copolymer of a vinyl-based monomer such as isobutylene, styrene, ethylene, or vinyl ether with an unsaturated carboxylic acid such as maleic anhydride or an anhydride thereof or a salt thereof, and the like), a polymer having hydrophilic substituents introduced therein (a polyester, polyamide, polystyrene, or a salt thereof, and the like into which a sulfo group, a carboxy group, a hydroxy group, or the like is introduced), and an aliphatic polyester-based resin [a polylactic acid-based resin (for example, polylactic acid)]. Furthermore, a resin that exhibits an adhesive function when softened by hot water or high-temperature steam and is a polyolefin-based resin, a polyester-based resin, a polyamide-based resin, a polyurethane-based resin, a thermoplastic elastomer, a rubber (styrene-based elastomer or the like), or the like can also be used as the thermal adhesive resin under moisture.

The thermal adhesive resin under moisture may be used alone, or two or more kinds of the thermal adhesive resin under moisture may be used in combination.

Preferable examples of the thermal adhesive resin under moisture include a vinyl alcohol-based polymer such as an ethylene-vinyl alcohol copolymer, a polylactic acid-based resin such as polylactic acid, and a (meth)acryl-based copolymer containing a (meth)acrylamide unit, and more preferably a vinyl alcohol-based polymer containing an $\alpha$-C2-10 olefin unit such as ethylene or propylene, and still more preferably an ethylene-vinyl alcohol-based copolymer.

The ethylene-vinyl alcohol-based copolymer can be obtained by saponifying the ethylene-vinyl ester-based copolymer.

Examples of vinyl ester monomers used for producing the ethylene-vinyl ester-based copolymer include vinyl formate, vinyl acetate, vinyl propionate, vinyl butyrate, vinyl isobutyrate, vinyl pivalate, vinyl versatate, vinyl caproate, vinyl caprylate, vinyl laurate, vinyl palmitate, vinyl stearate, vinyl oleate, and vinyl benzoate. Among them, vinyl acetate is most preferable from the viewpoint of costs.

The content (copolymerization ratio) of ethylene units in the ethylene-vinyl alcohol-based copolymer is, for example, 10 to 60 mol%, preferably 20 to 55 mol%, more preferably about 30 to 50 mol%. When the ethylene units are contained in the copolymer in this range, the copolymer has a unique property of having thermal adhesiveness under moisture but not having solubility in hot water. When the proportion of the ethylene units in the copolymer is too small, the ethylene-vinyl alcohol-based copolymer easily swells or gels under low temperature steam (water), and the form of the copolymer is easily changed only by wetting with water once. On the other hand, when the proportion of the ethylene units in the copolymer is too large, the copolymer has lowered hygroscopicity and hardly exhibits fiber adhesiveness by wet heat, making it difficult to produce a shaped product having practical strength. When the proportion of the ethylene units in the copolymer is particularly in the range of 30 to 50 mol%, the resin of the fiber is softened, and bonding points between the fibers are easily formed, so that the processability of the shaped product into a sheet or a plate-like structure is particularly excellent.

The saponification degree of vinyl alcohol units in the ethylene-vinyl alcohol-based copolymer is, for example, about 90 to 99.99 mol%, preferably about 95 to 99.98 mol%, and more preferably about 96 to 99.97 mol%. When the saponification degree is too small, the copolymer has lowered thermal stability, resulting in deterioration of stability due to thermal decomposition or gelation. On the other hand, when the saponification degree is too large, it is difficult to produce the fiber itself. As used herein, the saponification degree of vinyl alcohol units refers to the extent to which alkoxycarbonyl groups (also called ester groups, RO-CO-) in the vinyl alcohol units is hydrolyzed and replaced by hydroxy groups. The saponification degree is expressed as the percentage of hydroxy groups relative to the total number of carboxy groups and hydroxy groups. The saponification degree is determined by titration with sodium hydroxide.

The viscosity average polymerization degree of the ethylene-vinyl alcohol-based copolymer can be selected, if necessary, and is, for example, 200 to 2500, preferably 300 to 2000, more preferably about 400 to 1500. When the polymerization degree is in this range, the resulting fiber has an excellent balance between the spinnability and the adhesiveness. The viscosity average polymerization degree (P) is calculated from the viscosity ($\eta$) of an ethylene-vinyl alcohol-based copolymer using the following equation.

$$\log P = 1.613 \log\{\{\eta\} \times 10^4)/7.15\}$$

From the viewpoint of spinning, the melt index (MI) of the ethylene-vinyl alcohol-based copolymer is important. The MI indicates the flowability of a molten thermoplastic resin and refers to a typical index for quality control of thermoplastic resins. MI measurement is performed in accordance with JIS K7210. The MI of the ethylene-vinyl alcohol-based copolymer can be selected, if necessary. From the viewpoint of the balance between the spinnability and the adhesiveness of the resulting fiber, the MI of the ethylene-vinyl alcohol-based copolymer as measured under conditions of 190°C and a load of 2160 g is preferably 1.0 to 10 g/10 min, more preferably 1.3 to 7.0 g/10 min, and still more preferably 1.5 to 6.6 g/10 min.

The transverse cross-sectional shape (cross-sectional shape perpendicular to the length direction of the fiber) of the fiber containing an ethylene-vinyl alcohol-based copolymer (hereinafter, sometimes referred to as the present fiber) may be a round cross-sectional shape or a modified cross-sectional shape [flat, elliptical, polygonal, 3 to 14 lobed, T-shaped, H-shaped, V-shaped, dog-bone (I-shaped), or the like] which is a general solid cross-sectional shape, or may be a hollow cross-sectional shape.

The present fiber may be a composite fiber composed of a plurality of resins. The composite fiber may comprise an ethylene-vinyl alcohol-based copolymer on at least a part of the fiber surface. From the viewpoint of adhesiveness, it is preferable that the ethylene-vinyl alcohol-based copolymer continuously occupies at least a part of the fiber surface in the length direction.

Examples of the transverse cross-sectional structure of the composite fiber having a surface occupied by the ethylene-vinyl alcohol-based copolymer include structures of a core-sheath type structure, a sea-island type structure, a side-by-side type structure, a multilayer bonding type structure, a radial bonding type structure, and a random composite type structure. Among these cross-sectional structures, a core-sheath type structure in which the ethylene-vinyl alcohol-based copolymer continuously occupies the entire surface in the length direction (that is, a core-sheath type structure comprising a sheath part made of an ethylene-vinyl alcohol-based copolymer) is preferable from the viewpoint of high adhesiveness.

In the case of the composite fiber, two or more thermal adhesive resins under moisture may be combined, or a thermal adhesive resin under moisture and a non-thermal adhesive resin under moisture may be combined. As used herein, the term "non-thermal adhesive resin under moisture" refers to a resin that does not exhibit an adhesive function by either high-temperature steam or hot water.

Examples of the non-thermal adhesive resin under moisture include a water-insoluble or hydrophobic resin, specifically, a polyolefin-based resin, a (meth)acrylic resin, a vinyl chloride-based resin, a styrene-based resin, a polyester-based resin, a polyamide-based resin, a polycarbonate-based resin, a polyurethane-based resin, and a thermoplastic elastomer. The non-thermal adhesive resins under moisture may be used alone, or two or more kinds of the non-thermal adhesive resins under moisture may be used in combination. Among the non-thermal adhesive resins under moisture, a resin (for example, a polypropylene-based resin, a polyester-based resin, or a polyamide-based resin) having a melting point higher than that of a thermal adhesive resin under moisture (for example, an ethylene-vinyl alcohol-based copolymer) is preferable from the viewpoint of heat resistance and dimensional stability, and a polyester-based resin and a polyamide-based resin are more preferable from the viewpoint of excellent balance of heat resistance, fiber formability, and the like.

Examples of the polyester-based resin include preferably an aromatic polyester-based resin [polyethylene terephthalate-based resin such as polyethylene terephthalate (PET), polytrimethylene terephthalate, polybutylene terephthalate, polyethylene naphthalate, and the like] such as a poly C2-4 alkylene arylate-based resin, and more preferably a polyethylene terephthalate-based resin. The polyethylene terephthalate-based resin may contain, in addition to ethylene terephthalate units, units composed of other dicarboxylic acids [for example, isophthalic acid, naphthalene-2,6-dicarboxylic acid, phthalic acid, 4,4'-diphenylcarboxylic acid, bis(carboxyphenyl)ethane, 5-sodium sulfoisophthalic acid, or the like] or diols (for example, diethylene glycol, 1,3-propanediol, 1,4-butanediol, 1,6-hexanediol, neopentyl glycol, cyclohexane-1,4-dimethanol, polyethylene glycol, polytetramethylene glycol, or the like) at a ratio of about 20 mol% or less.

Examples of the polyamide-based resin include preferably an aliphatic polyamide such as polyamide 6, polyamide 66, polyamide 610, polyamide 10, polyamide 12, or polyamide 6-12 and a copolymer thereof, and a semi-aromatic polyamide synthesized from an aromatic dicarboxylic acid and an aliphatic diamine. The polyamide-based resin may contain units other than amide units.

In the case of a composite fiber composed of an ethylene-vinyl alcohol-based copolymer and a non-thermal adhesive resin under moisture (fiber-forming polymer), proportions (mass ratio) of the two components can be appropriately selected depending on the structure (for example, a core-sheath type structure). The mass ratio of the ethylene-vinyl alcohol-based copolymer/the non-thermal adhesive resin under moisture is, for example, 90/10 to 10/90, preferably 80/20 to 15/85, more preferably about 60/40 to 20/80. When the proportion of the ethylene-vinyl alcohol-based copolymer is too large, it is difficult to secure the strength of the fiber. When the proportion of the ethylene-vinyl alcohol-based copolymer is too small, it is difficult to make the ethylene-vinyl alcohol-based copolymer continuously present in the length direction of the fiber surface, resulting in deterioration of the adhesiveness. The same tendency is observed in the case of coating the surface of the non-thermal adhesive fiber under moisture with the ethylene-vinyl alcohol-based copolymer.

Examples of the composite fiber include preferably a composite fiber composed of an ethylene-vinyl alcohol-based copolymer and a polyester-based resin, and a composite fiber composed of an ethylene-vinyl alcohol-based copolymer and a polyamide-based resin, and more preferably a composite fiber composed of an ethylene-vinyl alcohol-based copolymer and a polyester-based resin.

Examples of the present fiber that is a core-sheath type composite fiber include a core-sheath type composite fiber comprising a sheath part composed of an ethylene-vinyl alcohol-based copolymer and a core part composed of a non-thermal adhesive resin under moisture, preferably a core-sheath type composite fiber comprising a sheath part composed of an ethylene-vinyl alcohol-based copolymer and a core part composed of a polyester-based resin, and a core-sheath type composite fiber comprising a sheath part composed of an ethylene-vinyl alcohol-based copolymer and a core part composed of a polyamide-based resin, and more preferably a core-sheath type composite fiber comprising a sheath part composed of an ethylene-vinyl alcohol-based copolymer and a core part composed of a polyester-based resin.

The average fineness of the present fibers is, for example, about 0.01 to 100 dtex, preferably 0.1 to 50 dtex, more preferably 0.5 to 30 dtex, and particularly preferably about 1 to 10 dtex. When the average fineness is in the above-mentioned range, the fiber has an excellent balance between the strength and the adhesiveness. The average fineness is measured by the method of JIS L 1015.

The average fiber length of the present fibers is, for example, about 10 to 100 mm, preferably 20 to 80 mm, more preferably 25 to 75 mm, and particularly preferably about 35 to 55 mm. When the average fiber length is in the above-mentioned range, the fibers are sufficiently entangled, thereby improving the mechanical strength of the shaped product. The average fiber length is measured by the method of JIS L 1015.

The crimping rate of the present fiber is, for example, about 1 to 50%, preferably 3 to 40%, more preferably 5 to 30%, and particularly preferably about 10 to 20%. The number of crimps of the present fiber is, for example, 1 to 100 crimps/inch, preferably 5 to 50 crimps/inch, more preferably about 10 to 30 crimps/inch. The crimping rate and the number of crimps are measured by the method of JIS L 1015.

The "shaped product made of a fiber containing an ethylene-vinyl alcohol-based copolymer, the shaped product having a nonwoven fiber structure" may further contain a non-thermal adhesive fiber under moisture. As used herein, the term "non-thermal adhesive fiber under moisture" refers to a fiber that does not exhibit an adhesive function by either high-temperature steam or hot water.

Examples of the non-thermal adhesive fiber under moisture include polyester-based fibers (an aromatic polyester fiber such as a polyethylene terephthalate fiber, a polytrimethylene terephthalate fiber, a polybutylene terephthalate fiber, or a polyethylene naphthalate fiber, and the like), polyamide-based fibers (an aliphatic polyamide-based fiber such as polyamide 6, polyamide 66, polyamide 11, polyamide 12, polyamide 610, or polyamide 612, a semi-aromatic polyamide-based fiber, an aromatic polyamide-based fiber such as polyphenylene isophthalamide, polyhexamethylene terephthalamide, and polyp-phenylene terephthalamide, and the like), polyolefin-based fibers (a poly C2-4 olefin fiber such as polyethylene and polypropylene), acryl-based fibers (an acrylonitrile-based fiber having an acrylonitrile unit such as an acrylonitrile-vinyl chloride copolymer, and the like), polyvinyl-based fibers (a polyvinyl acetal-based fiber and the like), polyvinyl chloride-based fibers (fibers of polyvinyl chloride, vinyl chloride-vinyl acetate copolymer, vinyl chloride-acrylonitrile copolymer, or the like), polyvinylidene chloride-based fibers (a vinylidene chloride-vinyl chloride copolymer fiber, a vinylidene chloride-vinyl acetate copolymer fiber, and the like), polyparaphenylene benzobisoxazole fibers, polyphenylene sulfide fibers, and cellulose-based fibers (for example, a rayon fiber, an acetate fiber, and the like). These non-thermal adhesive fibers under moisture may be used alone, or two or more kinds of the non-thermal adhesive fibers under moisture may be used in combination.

The average fineness and average fiber length of the non-thermal adhesive fibers under moisture are the same as those of the thermal adhesive fibers under moisture.

The proportions of the present fiber and the non-thermal adhesive fiber under moisture contained in the shaped product (the mass ratio of the present fiber/the non-thermal adhesive fiber under moisture) are, for example, 20/80 to 100/0, preferably 30/70 to 100/0. In the case of producing a hard shaped product, it is preferable that the proportion of the present fiber is larger, and the mass ratio of the present fiber/the non-thermal adhesive fiber under moisture is more preferably 80/20 to 100/0, still more preferably 90/10 to 100/0, and particularly preferably about 95/5 to 100/0. When the proportion of the present fiber is in the above-mentioned range, a shaped product capable of securing high surface hardness and bending behavior is obtained. For production of a shaped product that utilizes the property of the non-thermal adhesive fiber under moisture, the mass ratio of the present fiber/the non-thermal adhesive fiber under moisture is more preferably about 20/80 to 99/1, still more preferably about 30/70 to 90/10, and particularly preferably about 40/60 to 80/20.

The fiber-fiber bonding ratio of the shaped product is preferably 10 to 85%, more preferably 10 to 70%, still more preferably 10 to 60%, and particularly preferably about 10 to 35%. As used herein, the term "fiber-fiber bonding ratio" refers to a ratio of the number of fibers bonded in groups of two or more fibers to the total number of fibers in the cross section of a shaped product having a nonwoven fiber structure (that is, a fiber-fiber bonding ratio = 100 × the number of fibers bonded in groups of two or more fibers in the cross section of the shaped product/the total number of fibers in the cross section of the shaped product). Therefore, a low fiber-fiber bonding ratio means that the proportion of a plurality of fibers bonded to each other (the proportion of fibers being bundled and bonded) is small.

The fibers constituting the nonwoven fiber structure of the shaped product are bonded at contact points between the fibers. In order to exhibit a large bending stress with as few contact points as possible, it is preferable that the bonding points are uniformly distributed from the surface to the inside (center) and to the back surface of the shaped product along the thickness direction. When the bonding points are concentrated on the surface, the inside, or the like, not only it is difficult to ensure sufficient bending stress, but also shape stability in a portion having few bonding points is deteriorated.

Since the shaped product is formed of the present fibers, the shaped product is excellent in shape stability and handleability in cell culture. Since the shaped product can always maintain voids necessary for adhesion and proliferation of cells during a culture period, the shaped product has a high ability to maintain the morphology of cells in cell culture. Therefore, when the shaped product is used as a culture carrier, it is possible to immobilize cells, particularly adherent cells, and three-dimensionally culture the adherent cells.

In addition, the shaped product has an apparent density of, for example, 0.02 to 0.7 g/cm$^3$, preferably 0.025 to 0.3 g/cm$^3$, more preferably 0.03 to 0.15 g/cm$^3$, and still more preferably 0.075 to 0.125 g/cm$^3$ from the viewpoint of medium components (nutrient supply) for cell maintenance in cell culture, permeability of oxygen and the like, and waste removal. Cell proliferation can be continued for a long period of time by adjusting the apparent density to the above-mentioned range. As used herein, the "apparent density" is a value obtained by dividing the mass of a shaped product having a nonwoven fiber structure including voids by the volume of the shaped product. The apparent density can also be determined by measuring the basis weight (that is, the mass per unit area) and the thickness of the shaped product and dividing the basis weight by the thickness.

The shaped product is substantially composed of the present fibers without being impregnated with a resin. Further, the nonwoven fiber structure is formed by bonding of the present fibers. For example, the shaped product has a sufficiently large surface for cell adhesion and excellent shape stability when used for three-dimensional culture of adherent cells. Therefore, when using the shaped product as a carrier for cell culture in a bioreactor or the like, efficient cell proliferation can be achieved.

The shape of the shaped product is not particularly limited, and may be, for example, a spherical shape, a plate shape, a rod shape, a square shape, an elliptical shape, a disk shape, a cylindrical shape, a columnar shape, or the like. Among these shapes, a spherical shape and a disk shape are preferable. When the shaped product is used as a carrier for cell culture in a bioreactor, the shaped product is preferably spherical. Particularly, when used in a flowable bioreactor, a spherical carrier is more excellent in flowability than that of other shapes. When used in a fixed-bed bioreactor, the spherical carrier is easy to fill, has a small contact point between carriers, and is less likely to cause aggregation, and thus easy to handle.

When the shaped product has a disk shape, the size thereof is not particularly limited, and can be appropriately selected from the viewpoint of handleability, for example, with reference to the size of a culture plate that is usually distributed. On the other hand, when the shaped product is spherical, the average particle diameter thereof is preferably 1 mm or more, more preferably 2 mm or more, and still more preferably 3 mm or more from the viewpoint of facilitating separation and recovery of the carrier, and is preferably 20 mm or less, more preferably 10 mm or less, and still more preferably 6 mm or less from the viewpoint of increasing the cell culture surface area per unit volume. The average particle diameter is determined by measuring the maximum diameters of 10 carriers for cell culture randomly selected using a caliper and averaging them.

Since the shaped product has a nonwoven fiber structure in which the fibers are appropriately adhering, the shaped product has a high bending stress even when the shaped product is lightweight with a low density. This shaped product has air permeability and heat insulation properties, high hardness, and excellent folding resistance and toughness. Specifically, the shaped product is formed into a plate shape and is hardly locally deformed even when a load is applied to the surface, and is curved and deformed relatively to the applied stress to absorb the stress, and thus has high impact resistance, and is not easily broken or fractured even when a strong impact is applied. Furthermore, since the shaped product can be substantially composed of only fibers and does not require addition of a chemical binder or a special agent, the shaped product is inert to cells without using a component that generates harmful components (volatile organic compounds such as formaldehyde).

Components other than the constituent fibers that adhere to the shaped product may be removed by washing or the like. An example of the component includes a fiber finish. The fiber finish is used to suppress friction of fibers during a step of entangling fibers in a general method for producing a nonwoven fiber structure. A method for removing the component is not particularly limited, and for example, the shaped product may be washed by using a liquid component including water, an organic solvent, an acid or alkali component, a surfactant, or the like. A method for washing the shaped product is also not particularly limited, and for example, the shaped product may be washed by immersion in the liquid component. For continuous washing, the liquid component may be passed through the inside of the shaped product while the shaped product is conveyed on a mesh conveyor. The washing may be performed a plurality of times. The washing may be performed stepwise with different liquid components. The shaped product may be finally washed with water one or more times so that the washing component does not remain in the shaped product. After washing, the liquid component may be removed by suction, compression or the like, and the liquid remaining in the shaped product may be evaporated and dried by hot air or the like.

When the shaped product is used for cell culture for a long period of time, a difference in cell density occurs between the central part and the outer edge part of the shaped product as cell proliferation continues for a long period of time. In order to avoid such uneven cell proliferation, one or more through holes may be provided in the shaped product. The presence of through holes enables efficient nutrient supply to the inside (central part) of the shaped product and waste removal, and thus the proliferation of cells in the shaped product becomes uniform. The proliferation of cells is also observed in the through holes. The size and number of the through holes provided in the shaped product may be appropriately determined depending on the size of the shaped product, and are not particularly limited. Depending on the size of the shaped product, for example, 1 to 4 through holes, preferably 1 or 2 through holes of about 0.5 mm to 2 mm, preferably about 0.7 $\mu$m to 1.5 mm may be provided.

The shaped product is produced by forming a nonwoven fiber structure using the present fibers. For such production, any method known in the art may be used. For example, when the present fiber is a thermal adhesive fiber under moisture, the shaped product can be produced by the following method. First, fibers including the present fibers are formed into a web. As a method for forming the web, a conventional method, for example, a direct method such as a spunbond method or a melt blow method, a carding method using staple fibers or the like, a dry method such as an air lay method, or the like can be used. Among these methods, in particular, a carding method using staple fibers is widely used. Examples of the web obtained using staple fibers include a random web, a semi-random web, a parallel web, and a crosslap web. Among these webs, semi-random webs and parallel webs are preferable for increasing the proportion of bundled fusion fibers. Next, the fiber web thus obtained is sent to the next step by a mesh conveyor, and then exposed to superheated steam or high-temperature steam (high-pressure steam) flow, thereby obtaining a shaped product having a nonwoven fiber structure. Specifically, when the fiber web conveyed by the mesh conveyor passes through the high-temperature steam flow ejected from a nozzle of a steam injecting apparatus, the fibers three-dimensionally adhere to each other by the sprayed high-temperature steam.

Basically, a belt conveyor to be used is not particularly limited as long as the fiber web to be processed can be subjected to high-temperature steam treatment while being compressed to a desired density. An endless mesh conveyor is suitably used. A general single mesh conveyor may be used, or if necessary, two mesh conveyors may be used in combination to convey the web sandwiched between the mesh conveyors. Conveying the web in such a manner during treatment can suppress deformation of the web caused by external forces such as water and high-temperature steam used in the treatment and vibrations of the mesh conveyors. In addition, the density and thickness of the shaped product having a nonwoven fiber structure after the treatment can be controlled by adjusting an interval between the belts.

When two mesh conveyors are used in combination, a steam injecting apparatus for supplying steam to a web is mounted on one of the mesh conveyors and supplies steam to the web through a mesh structure of the conveyor. A suction box may be mounted on the other mesh conveyor. The suction box allows excess steam that has passed through the web to be sucked and discharged. In order to treat both the front and back sides of the web with steam simultaneously, a suction box may be additionally mounted on a downstream part of the one mesh conveyor having the steam injecting apparatus, and a steam injecting apparatus may be installed in a downstream part of the other mesh conveyor having the suction box. When there are no steam injecting apparatus and suction box in the downstream parts, the front and back of the fiber web treated once are reversed and passed through the treatment apparatus again, so that the front and back of the fiber web can be treated with steam.

The shape of the mesh conveyor is not particularly limited as long as it does not hinder the conveyance and high-temperature steam treatment of the web. However, when the high-temperature steam treatment is performed, the surface shape of the belt may be transferred to the surface of the fiber web depending on the conditions, and thus it is preferable to appropriately select the shape of the mesh conveyor depending on the intended use. Particularly, when it is desired to obtain a shaped product having a flat surface, a net having a fine mesh may be used. However, the upper limit is about 90 mesh. A net finer than about 90 mesh has low air permeability, and is difficult for steam to pass through. From the viewpoint of heat resistance to steam treatment, preferable examples of materials of the mesh conveyor include a metal, and a heat-resistant resin, such as a polyester-based resin subjected to heat treatment, a polyphenylene sulfide-based resin, a polyarylate-based resin (wholly aromatic polyester-based resin), and an aromatic polyamide-based resin.

Since the high-temperature steam injected from the steam injecting apparatus is an air flow, unlike water flow entanglement treatment or needle punch treatment, the high-temperature steam enters the inside of the web without greatly moving the fibers in the web as the workpiece. It is presumed that the penetration of the steam flow into the web and the humid heat effect allow the steam flow to efficiently cover the surface of each fiber present in the web under humid heat conditions, thereby enabling uniform thermal adhesion. Since this treatment is performed under a high-speed air flow in an extremely short time, though heat conduction from the steam to the fiber surfaces is sufficient, the treatment is completed before the heat can sufficiently conduct to the inside of the fiber. Therefore, deformation such as compression or loss of thickness of the entire fiber web to be treated due to the pressure or heat of the high-temperature steam is less likely to occur. As a result, humid-heat adhesion is completed so that the degree of adhesion in the surface and thickness directions is substantially uniform without causing significant deformation in the fiber web.

Furthermore, for obtaining a shaped product having high surface hardness and bending strength, it is important to expose the web to be treated to high temperature steam in a state of being compressed to a desired apparent density between mesh conveyors or rollers when the high-temperature steam is supplied to the web for treatment. Particularly, in order to obtain a relatively high-density shaped product, it is necessary to compress the fiber web at a sufficient pressure when the fiber web is treated with high-temperature steam. Furthermore, securing an appropriate clearance between the mesh conveyors or the rollers allows the thickness or density to be adjusted to the desired value. In the case of the mesh conveyor, since it is difficult to compress the web at once, it is preferable to set the tension of the mesh conveyor as high as possible and gradually narrow the clearance from the upstream of the steam treatment point. Further, the web is processed into a shaped product having the desired bending hardness, surface hardness, lightness, and air permeability by adjusting the steam pressure and the treatment speed.

When higher hardness is desired, the back side of the mesh conveyor opposite the nozzle across the web may be made of a stainless plate or the like so as to have a structure that prevents the passage of steam. In this case, steam that has passed through the web to be treated is reflected by the plate or the like, and the present fibers are firmly bonded by the heat-retaining effect of the steam. Conversely, when slight bonding is required, a suction box may be disposed to discharge excess steam to the outside of the room.

As the nozzle for injecting the high-temperature steam, a plate or a die in which predetermined orifices are continuously arranged in the width direction may be used, and the plate or the die may be placed such that the orifices are arranged in the width direction of the web supplied. The number of orifice rows may be one or more, and a plurality of orifice rows may be arranged in parallel. Further, a plurality of nozzle dies, each having a row of orifice rows may be installed in parallel.

When a nozzle of a type in which an orifice is opened in a plate is used, the thickness of the plate may be, for example, about 0.5 to 1 mm. The diameter and pitch of orifices are not particularly limited as long as the desired fiber fixing is possible. For example, the diameter of orifices is usually 0.05 to 2 mm, preferably 0.1 to 1 mm, and more preferably about 0.2 to 0.5 mm. The pitch between orifices is usually 0.5 to 3 mm, preferably 1 to 2.5 mm, more preferably about 1 to 1.5 mm. When the diameter of orifices is too small, problems related to equipment that the processing accuracy of the nozzle becomes low and processing is difficult, as well as a problem in operation that clogging easily occurs arise. Conversely, when the diameter of orifices is too large, the steam injection force decreases. On the other hand, when the pitch is too small, the nozzle holes become too closely spaced, so that the strength of the nozzle itself decreases. On the other hand, when the pitch is too large, high-temperature steam may not be sufficiently applied to the entire surface of the web, so that bonding between fibers in the web becomes uneven, and the strength of the shaped product decreases.

The high-temperature steam is not particularly limited as long as the intended fiber can be fixed. Depending on the material and form of the fiber used, the pressure is set at, for example, 0.05 to 2 MPa, preferably 0.05 to 1.5 MPa, and more preferably about 0.1 to 1 MPa. When the pressure of the steam is too high, fibers forming the web may move to cause disturbance of the formation, or the fibers may be too melted to partially retain the fiber shape. When the pressure is too low, the amount of heat required for fusing the fibers cannot be applied to the web, or the steam cannot pass through the web, so that fiber fusion unevenness may occur in the thickness direction, and it may be difficult to control uniform ejection of steam from the nozzle.

The temperature of the high-temperature steam is, for example, 70 to 150°C, preferably 80 to 120°C, and more preferably about 90 to 110°C. The treatment speed of the web with high-temperature steam is, for example, 200 m/min or less, preferably 0.1 to 100 m/min, and more preferably about 1 to 50 m/min.

If necessary, it is possible to impart a predetermined concavo-convex shape or the like to the mesh conveyor and transfer the concavo-convex shape or the like from the mesh conveyor to a board product obtained. In addition, a laminate may be formed by laminating with another material, or may be processed into a desired form (various shapes such as a columnar shape, a quadrangular prism shape, a spherical shape, and an ellipsoidal shape) by molding processing.

After the fibers of the fiber web partially undergo humid-heat adhesion in this manner, since moisture may remain in the resulting shaped product, the web may be dried, if necessary. Regarding drying, it is necessary that the surface of the shaped product in contact with a drying heater does not lose its fiber form due to melting of the fiber or the like after drying, and a conventional method can be used as long as the fiber form can be maintained. For example, a large drying facility such as a cylinder dryer or a tenter used for drying a nonwoven fabric may be used. Since residual moisture is usually very small and often at a level that can be dried by a relatively mild drying means, a non-contact method such as far-infrared irradiation, microwave irradiation, or electron beam irradiation, a method using hot air or the like is preferable.

Furthermore, the shaped product may be obtained by bonding the present fibers with high-temperature steam as described above, or may also be obtained by bonding the present fibers by other conventional processing methods, for example, including thermal pressure fusion (such as thermal embossing) and mechanical compression (such as needle punching). The other conventional methods may be used for bonding the obtained shaped products to each other.

When the present fiber is a heat-adhesive fiber under moisture, the fibers are bonded also by immersing the fiber web in hot water. However, in a method comprising immersion in hot water, it is difficult to control the fiber-fiber bonding ratio and to obtain a shaped product having high uniformity of the fiber-fiber bonding ratio. It is presumed that the causes are different humid-heat adhesiveness depending on positions due to the influence of air inevitably contained in the fiber web, the effect of the air pushed out of the fiber web on the structure, the deformation of the microstructure inside the fiber due to a take-up roller when the fiber web subjected to humid-heat adhesion is taken out from the hot water, differences in the deformation of the microstructure in the vertical direction due to the weight of the hot water contained in the taken-out fiber web, and the like.

The shaped product thus obtained can be further processed into a desired shape by, for example, a known method (for example, press or cutting).

In the production method of the present invention, adherent cells are cultured using the shaped product as a cell culture carrier. Specifically, adherent cells are supported on the shaped product while being cultured. The support of cells may be carried out by any method as long as the cells are finally supported on the shaped product. Since the cells are adherent cells, the cells easily adhere to the shaped product by contacting the cells with the shaped product, and are thus supported on or in the shaped product. For example, the adherent cells may be supported on or in the shaped product by a method comprising dropping a cell suspension onto the shaped product, a method comprising immersing the shaped product in a cell suspension, or the like. The cell suspension may be a cell suspension in a liquid, for example, water or a culture medium at a concentration of preferably about $10^3$ to $10^8$ cells/ml, more preferably $10^3$ to $10^6$ cells/ml. The adherent cells are preferably supported within voids among fibers of the shaped product.

The adherent cells thus supported on the shaped product are then cultured under stirring. For culturing, culture conditions including a culture medium and a culture temperature suitable for cells to be cultured (that is, the adherent cells supported on the shaped product) may be selected. Examples of the culture conditions include the conditions described in "Handbook of Cultured Cells: Fundamentals and Analytical Methods of Cell Culture", Toshio Kuroki, edited by Namho Huh, YODOSHA, published in July 2004, and the like.

As the culture medium, a liquid medium is preferably used, and a conventional medium suitable for the adherent cells to be cultured may be used. For example, when animal cells are cultured, a medium containing various components including various essential amino acids, various vitamins, and saccharides such as glucose (for example, Dulbecco's Modified Eagle's Medium or the like) may be used. The contents of the components in a culture medium may be amounts suitable for culturing the cells. Furthermore, the medium may be, for example, a fetal bovine serum medium, a serum-free medium, a human serum medium, or the like.

Stirring may be performed using, for example, a stirrer or a shaker. The stirring speed is, for example, about 30 rpm to 140 rpm, preferably about 50 rpm to 90 rpm. The stirring is performed at least in a part of the culture period, preferably throughout the entire culture period.

In the production method of the present invention, stirring promotes the aggregation of adherent cells in the shaped product, thereby efficiently forming a cell aggregate. In addition, stirring causes the cell aggregate formed in the shaped product to separate from the shaped product and be released into the medium. The cell aggregate thus produced can be easily collected from the medium by, for example, a method such as filtration.

Hereinafter, the present invention will be explained in more detail with reference to Examples and Comparative Examples, to which the present invention is not limited.

EXAMPLES

Example 1: Production of Cell Aggregate by Production Method of Present Invention -1

(1) Preparation of Shaped product

A shaped product in the form of a disk, having an apparent density of 0.1 g/cm³, a thickness of 2 mm, and a diameter of 6 mm was prepared. The shaped product was provided with two through holes having a diameter of about 1 mm. A carded web having a basis weight of about 100 g/m² was produced by a carding method using core-sheath type composite staple fibers ("SOFISTA" manufactured by Kuraray Co., Ltd., average fineness: 1.7 dtex, average fiber length: 51 mm, mass ratio of ethylene-vinyl alcohol-based copolymer/non-thermal adhesive resin under moisture = 50/50, number of crimps: 22.9 crimps/inch, crimping rate: 12.2%) in which the core component was polyethylene terephthalate and the sheath component was an ethylene-vinyl alcohol copolymer [ethylene content: 44 mol%, saponification degree: 98.4 mol%, MI (temperature: 190°C, load: 2160 g) 5.5 g/10 min] as the present fibers, and 7 sheets of these webs were stacked to obtain a carded web having a total basis weight of 700 g/m². The carded web thus obtained was transferred to a mesh conveyor equipped with a stainless endless net of 50 mesh and 500 mm width.

The mesh conveyor consists of a pair of conveyors, a lower conveyor and an upper conveyor, and a steam injection nozzle is installed on the back side of the belt of each of the conveyors, so that high-temperature steam can be injected through the belt onto the web passing between the conveyors. Furthermore, each of the conveyors is equipped with a metal roll for adjusting the web thickness (hereinafter, sometimes abbreviated as a "web thickness adjustment roll") on the upstream side of the nozzle. The lower conveyor has a flat upper surface (that is, the surface through which the web passes). On the other hand, the upper conveyor has a lower surface that is curved along the web thickness adjustment roll. The web thickness adjustment roll of the upper conveyor is disposed to form a pair with the web thickness adjustment roll of the lower conveyor. The upper conveyor is movable up and down, so that an interval between the web thickness adjustment rolls of the upper conveyor and the lower conveyor can be adjusted to a predetermined interval. Further, the upstream side of the upper conveyor is inclined at an angle of 30 degrees relative to the downstream part using the web thickness adjustment roll as a reference point (with respect to the lower surface on the downstream side of the upper conveyor), and the downstream part is bent so as to be arranged in parallel with the lower conveyor. When the upper conveyor moves up and down, the upper conveyor moves while maintaining this parallel relationship with the lower conveyor. These mesh conveyors are designed to rotate in the same direction at the same speed, so that the web can be pressurized while the conveyor belts of the both mesh conveyors and the web thickness adjustment rolls of the both mesh conveyors maintain a predetermined clearance. This is for adjusting the web thickness before steam treatment by operating like a so-called calendering process. Specifically, the carded web fed from the upstream side travels on the lower conveyor, and the interval between the carded web and the upper conveyor gradually decreases until the carded web reaches the web thickness adjustment roll. When the interval becomes narrower than the web thickness, the web is sandwiched between the upper and lower conveyor belts and travels while being gradually compressed. The web is compressed to a thickness almost equal to the clearance provided between the web thickness adjustment rolls, and is subjected to the steam treatment in the state of such a thickness. Then the web travels in the downstream part of the conveyor while maintaining the thickness. In this Example, the web thickness adjustment roll was adjusted so as to have a linear pressure of 50 kg/cm.

Next, the carded web was introduced into a steam injecting apparatus provided on a lower conveyor, and high-temperature steam of 0.4 MPa was jetted (vertically) from the apparatus so as to pass in the thickness direction of the carded web to perform steam treatment, thereby obtaining a shaped product having a nonwoven fiber structure. This steam injecting apparatus comprised nozzles installed in a lower conveyor so as to blow high-temperature steam toward a web via a conveyor net, and a suction device installed in an upper conveyor. In addition, on the downstream side of the injecting apparatus in the web traveling direction, another steam injecting apparatus in which the arrangement of the nozzles and the suction device was reversed was installed, and steam treatment was thus applied to both the front and back surfaces of the web.

A steam injecting apparatus was used in which steam jet nozzles having an orifice diameter of 0.3 mm were arranged in a single row at a pitch of 1 mm along the width direction of the conveyor. The processing speed was 3 m/min, and the interval (distance) between the upper and lower conveyor belts on the nozzle side and the suction side was 10 mm. The nozzles were disposed on the back side of the conveyor belt so as to be almost in contact with the belt.

The shaped product thus obtained had a board-like form, was very hard as compared with a general nonwoven fabric, and was excellent in shape stability. The fiber adhesion ratio of the obtained shaped product was 26.7%. The shaped product thus obtained was processed into a plate having a thickness of 2 mm, and further punched out into a disk having a diameter of 6 mm.

Two through holes each having a diameter of about 1 mm were provided in the obtained disk-shaped product.

(2) Cell Culture under Stirring Using Shaped product

Cells were seeded on the shaped product prepared in (1). As the cells, human immortalized adipose derived mesenchymal stem cells [ADSC (ASC52telo)] were used. The cells were suspended in a medium to prepare a cell suspension of $6 \times 10^6$ cells/mL. The cells were seeded on the shaped product by adding dropwise 45 $\mu$L of the cell suspension per shaped product with a micropipette. The ADSCs seeded on the shaped product were cultured in a spinner flask (medium amount: 50 mL) under stirring (70 rpm) at 37°C for 20 days. As the medium, a Dulbecco's Modified Eagle's Medium (DMEM) supplemented with 10% FBS, 50 U/mL penicillin, and 50 $\mu$g/mL streptomycin was used. Half the volume of medium was changed at a frequency of twice every 7 days.

(3) Cell Aggregate

After the cell culture described in (2), formation of cell aggregates was observed. Granular cell aggregates suspended in the medium were observed. The cell aggregates were collected by filtration. The size of the obtained cell aggregates was measured by dimension measurement in a micrograph. As a result, the obtained cell aggregates had a diameter or major diameter of about 2 mm.

Example 2: Production of Cell Aggregate by Production Method of Present Invention -2

(4) Preparation of Shaped product

A shaped product in the form of a disk, having no through hole was prepared by the method described in Example 1 (1).

(5) Cell Culture under Stirring Using Shaped product

Cells were seeded on the shaped product prepared in (4). As the cells, human immortalized adipose derived mesenchymal stem cells (ADSCs) were used. The cells were suspended in a medium to prepare a cell suspension of $6 \times 10^6$ cells/mL. The cells were seeded on the shaped product by adding dropwise 45 μL of the cell suspension per shaped product with a micropipette. The ADSCs seeded on the shaped product were cultured in a spinner flask (medium amount: 50 mL) under stirring (70 rpm) at 37°C for 20 days. As the medium, a Dulbecco's Modified Eagle's Medium (DMEM) supplemented with 10% FBS, 50 U/mL penicillin, and 50 μg/mL streptomycin was used. Half the volume of medium was changed at a frequency of twice every 7 days.

(6) Cell Aggregate

After the cell culture described in (5), formation of cell aggregates was observed. Granular cell aggregates suspended in the medium were observed. The cell aggregates were collected by filtration. The size of the obtained cell aggregates was measured by dimension measurement in a micrograph. As a result, the obtained cell aggregates had a diameter or major diameter of about 2 mm.

Comparative Example 1: Production of Cell Aggregate by Cell Culture without Stirring

Cells were seeded on the shaped product prepared in Example 1 in the same manner as in Example 1. ADSCs were used as the cells. The ADSCs seeded on the shaped product were cultured in a 12 well dish (medium: 1 mL) at 37°C for 20 days under static conditions. A Dulbecco's Modified Eagle's Medium (DMEM) supplemented with 10% FBS, 50 U/mL penicillin, and 50 μg/mL streptomycin was used. Half the volume of medium was changed at a frequency of twice every 7 days. After 20 days of cell culture, formation of cell aggregates was not observed.

Comparative Example 2: Preparation of Cell Aggregate by Conventional Method -1

ADSCs were suspended in a medium to prepare a cell suspension of $2.5 \times 10^5$ cells/mL. As the medium, a Dulbecco's Modified Eagle's Medium (DMEM) supplemented with 10% FBS, 50 U/mL penicillin, and 50 μg/mL streptomycin was used. In a 5 mL centrifuge tube, 1 mL of the cell suspension was placed and was cultured at 37°C for 45 days. Half the volume of medium was changed at a frequency of twice every 7 days. After 45 days of cell culture, formation of cell aggregates was observed. The size of the obtained cell aggregates was measured in the same manner as in Example 1. As a result, the obtained cell aggregates had a diameter or major diameter of about 1.5 mm.

Comparative Example 3: Preparation of Cell Aggregate by Conventional Method -2

ADSCs were suspended in a medium to prepare a cell suspension of $1.0 \times 10^4$ cells/mL. As the medium, a Dulbecco's Modified Eagle's Medium (DMEM) supplemented with 10% FBS, 50 U/mL penicillin, and 50 μg/mL streptomycin was used. In a 96 well U-bottom plate, 1 mL of the cell suspension was placed and cultured at 37°C for 2 days. After 2 days of cell culture, formation of cell aggregates was observed. The size of the obtained spheroids was measured in the same manner as in Example 1. As a result, the obtained cell aggregates had a diameter or major diameter of about 500 μm.

Evaluation of Cell Viability in Cell Aggregate

In the cell aggregates obtained in Example 1 and the cell aggregates obtained in Comparative Example 2, cell viability was evaluated. For the evaluation, Live-Dead Assay by confocal laser microscope was performed. Evaluation was performed using a Live or DeadTM (registered trademark) cell viability assay kit (manufactured by Thermo Fisher Scientific). The kit uses two dyes, Calcein AM (for living cells) and a non-cell penetrating DNA binding dye. The Calcein AM is non-fluorescent, and produces hydrophilic Calcein that is apt to accumulate in cytoplasm when hydrolyzed by an endogenous esterase. The esterase activity is proportional to the number of living cells. The DNA-binding dye does not penetrate live cells having intact membranes and binds only to DNA of dead cells. Accordingly, Live/Dead is evaluated by green/red.

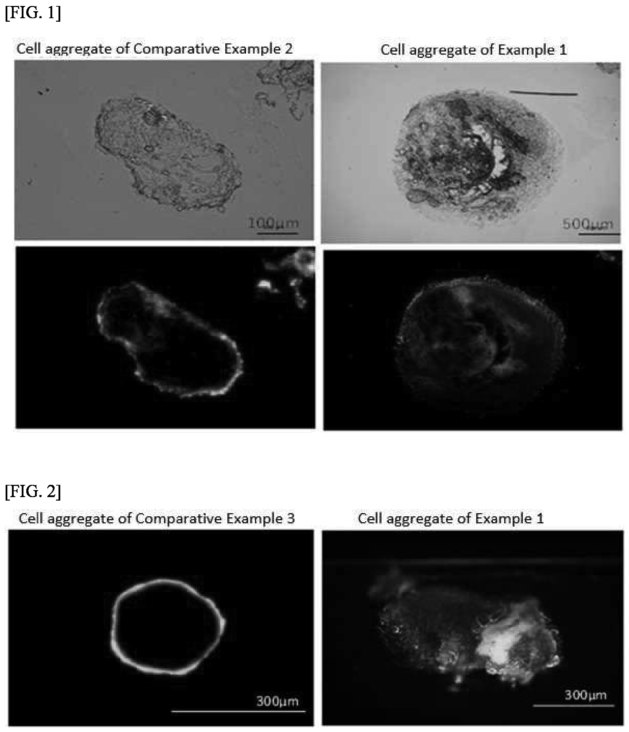

As a result, in the cell aggregates obtained in Example 1, survival of internal cells was observed, whereas in the cell aggregates obtained in Comparative Example 2, survival of internal cells was not observed (Fig. 1). This is considered to be because the shaped product containing thermal adhesive fibers under moisture and having a nonwoven fiber structure has moderate hydrophilicity, so that the production of the extracellular matrix proceeds, thereby suppressing excessive confluence of cells and improving the viability.

Evaluation of Cell Viability in Cell Aggregates after Freezing and Thawing

In the cell aggregates obtained in Example 1 and the cell aggregates obtained in Comparative Example 3, cell viability after freezing and thawing was evaluated. The cell aggregates were rinsed with PBS (phosphate buffered saline), placed in a microtube containing 1 mL of a cryopreservative CELLBANKER 1 (manufactured by Zenogen Pharma Co., Ltd.), and then shaken for 30 seconds on an orbital shaker. The CELLBANKER 1 was removed from the microtube, and the same amount of fresh CELLBANKER 1 was added to the microtube. The microtube was placed in a cell freezing container, Mr. Frosty (manufactured by Thermo Fisher Scientific) and the cell aggregates were frozen in a deep freezer at -90°C for 24 hours. Then, the microtube was held in liquid nitrogen for 24 hours. The microtube was removed from the liquid nitrogen, and placed in a water bath at 37°C to thaw the cell aggregates. The cell aggregates were removed from the microtube and subjected to Live-Dead Assay as described above.

As a result, in the cell aggregates obtained in Example 1, survival of internal cells was observed even after freezing and thawing (Fig. 2). On the other hand, in the cell aggregates obtained in Comparative Example 3, survival of internal cells was not observed (Fig. 2). Therefore, it was found that the cell aggregates of the present invention had improved cell viability after freezing and thawing as compared with conventional cell aggregates. Usually, freezing leads to cell death due to disruption of the cell membrane by ice formation. In the cell aggregates of the present invention, however, it is considered that the presence of extracellular matrix inhibits ice growth, thereby reducing damage to the cells.

Confirmation of Extracellular Matrix in Cell Aggregate

The cell aggregates obtained in Example 1 and the cell aggregates obtained in Comparative Example 2 were subjected to picro-Sirius red staining. Dyeing was performed using a picro-Sirius red staining kit (manufactured by ScyTek Laboratories). In the picro-Sirius red staining, collagen is stained red, and cytoplasm and other proteins are stained yellow. After dyeing, a red region and a yellow region were visually identified, and the area of each region was analyzed using image analysis software WinROOF (manufactured by MITANI CORPORATION).

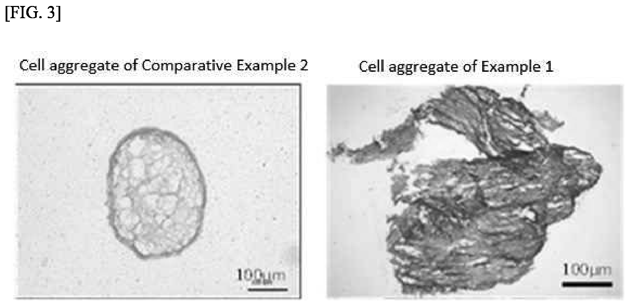

As a result, it was found that in the cell aggregates obtained in Example 1, the proportion of the area stained red was 45.3%, and components other than collagen were present at a proportion of about 50% or more of the whole (Fig. 3). On the other hand, in the cell aggregates of Comparative Example 2, almost 100% of the whole was stained red, indicating that collagen was the main component of the extracellular matrix (Fig. 3).

**Claims**

1. A cell aggregate comprising adherent cells and extracellular matrix molecules, the cell aggregate comprising viable cells even at a depth of 30 $\mu$m or more

from a surface of the cell aggregate.

2. The cell aggregate according to claim 1, comprising viable cells even at a depth of 30 $\mu$m or more from the surface of the cell aggregate after freezing and thawing the cell aggregate.

3. The cell aggregate according to claim 1 or 2, which is granular with a diameter of more than 1.5 mm.

4. The cell aggregate according to any one of claims 1 to 3, wherein less than 50% of the extracellular matrix molecules is collagen.

5. The cell aggregate according to any one of claims 1 to 4, which is in a frozen state.

6. A method for producing a cell aggregate, the method comprising:

   supporting an adherent cell on a shaped product made of a fiber containing an ethylene-vinyl alcohol-based copolymer, the shaped product having a nonwoven fiber structure; and culturing the adherent cell under stirring.

7. The method according to claim 6, wherein the fiber containing an ethylene-vinyl alcohol-based copolymer is a thermal adhesive fiber under moisture.

8. The method according to claim 6, wherein the fiber containing an ethylene-vinyl alcohol-based copolymer is a composite fiber containing a non thermal adhesive fiber under moisture.

9. The method according to claim 8, wherein the composite fiber is a core-sheath type composite fiber comprising a sheath part comprising an ethylene-vinyl alcohol-based copolymer and a core part comprising a polyester-based resin.

10. The method according to any one of claims 6 to 9, comprising:

    supporting the adherent cell on the shaped product having an apparent density of 0.02 to 0.7 g/cm$^3$: and
    culturing the adherent cell under stirring at 30 to 140 rpm.

EP 4 779 014 A1

[FIG. 1]

Cell aggregate of Comparative Example 2 | Cell aggregate of Example 1

[FIG. 2]

Cell aggregate of Comparative Example 3 | Cell aggregate of Example 1

15

[FIG. 3]

Cell aggregate of Comparative Example 2

Cell aggregate of Example 1

## INTERNATIONAL SEARCH REPORT

| | International application No. |
|---|---|
| | **PCT/JP2024/032716** |

**A. CLASSIFICATION OF SUBJECT MATTER**

*C12N 5/00*(2006.01)i
FI:   C12N5/00

According to International Patent Classification (IPC) or to both national classification and IPC

**B. FIELDS SEARCHED**

Minimum documentation searched (classification system followed by classification symbols)

C12N5/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | WO 2017/159862 A1 (KYOTO UNIVERSITY) 21 September 2017 (2017-09-21) claims, paragraphs [0077]-[0081], fig. 1A, B | 1-5 |
| A | | 6-10 |
| X | JP 2023-36560 A (TOYO INK SC HOLDINGS CO., LTD.) 14 March 2023 (2023-03-14) claims, paragraphs [0160]-[0164], table 9 | 1, 2, 4, 5 |
| A | | 3, 6-10 |
| X | WO 2021/100829 A1 (SUMITOMO DAINIPPON PHARMA CO., LTD.) 27 May 2021 (2021-05-27) claims (particularly, claims 21, 23) | 1, 2, 4, 5 |
| A | | 3, 6-10 |
| X | US 2014/0051168 A1 (VUKASINOVIC, Jelena) 20 February 2014 (2014-02-20) claims, paragraphs [0047], [0048], [0148]-[0151] | 6, 7 |
| A | | 1-5, 8-10 |

☐ Further documents are listed in the continuation of Box C.     ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **19 November 2024** | **03 December 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT
### Information on patent family members

International application No.

**PCT/JP2024/032716**

| Patent document cited in search report | | | Publication date (day/month/year) | Patent family member(s) | | | Publication date (day/month/year) |
|---|---|---|---|---|---|---|---|
| WO | 2017/159862 | A1 | 21 September 2017 | US | 2020/0325448 | A1 | |
| | | | | claims, paragraphs [0194]-[0198], fig. 1 | | | |
| | | | | EP | 3431584 | A1 | |
| | | | | CN | 109153973 | A | |
| JP | 2023-36560 | A | 14 March 2023 | (Family: none) | | | |
| WO | 2021/100829 | A1 | 27 May 2021 | US | 2023/0000071 | A1 | |
| | | | | claims | | | |
| | | | | EP | 4063496 | A1 | |
| | | | | KR | 10-2022-0104764 | A | |
| | | | | CN | 115175989 | A | |
| US | 2014/0051168 | A1 | 20 February 2014 | (Family: none) | | | |

Form PCT/ISA/210 (patent family annex) (July 2022)

# EP 4 779 014 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 7105487 B

**Non-patent literature cited in the description**

- **TOSHIO KUROKI**. Handbook of Cultured Cells: Fundamentals and Analytical Methods of Cell Culture. July 2004